# EUROPEAN PATENT SPECIFICATION

(11) **EP 2 620 180 B1**
(45) Date of publication and mention of the grant of the patent: **04.05.2016**
(21) Application number: 13151690.8
(22) Date of filing: 17.01.2013
(51) Int. Cl.: A61N 1/375, H01R 43/20, H01R 43/02, H01R 13/52, H01R 24/58

(54) **Method for assembling a feed-through connector assembly in an implantable pulse generator**
Verfahren zur Anordnung einer Durchführungssteckeranordnung in einem implantierbaren Impulserzeuger
Procédé d'assemblage d'un connecteur dans un générateur d'impulsion implantable

(30) Priority: 27.01.2012 US 201213359954
(43) Date of publication of application: 31.07.2013
(73) Proprietor: QIG Group, LLC, Clarence, New York 14031 (US)
(72) Inventor: Kelsch, Daniel N., Fairview Park, Ohio 44126 (US); Smith, Alexander K., Chesterland, Ohio 44026 (US)
(74) Representative: Lecomte & Partners

(56) References cited:
- US-A1- 2003 040 780
- US-A1- 2007 202 728
- US-A1- 2008 071 320
- US-A1- 2012 253 424

## Description

### FIELD OF THE INVENTION

This application relates generally to a connector for a medical device and, more specifically, to a feed-through connector assembly for connecting to a connector assembly for achieving electrical contact with an Internal Pulse Generator.

### BACKGROUND OF THE INVENTION

Medical devices have been implanted in patients to perform a variety of tasks. For example, programmable pulse generating systems are used to treat chronic pain by providing electrical stimulation pulses from an epidural electrode array placed near a patient's spine. Such Spinal Cord Stimulation (SCS) is useful for reducing pain in certain populations of patients. SCS systems typically include one or more electrodes connected to one or more connectors of an External Pulse Generator (EPG) or an Implanted Pulse Generator (IPG) via leads. In the case of an EPG, the lead must be connected to the EPG via an exit from the body. The pulse generator, whether internal or external, generates pulses that are typically delivered to the dorsal column fibers within the spinal cord through the electrodes which are implanted along or near the dura of the spinal cord. In a typical situation, the attached leads exit the spinal cord and are tunneled around the torso of the patient to a subcutaneous pocket where the IPG is implanted, or the wires exit the patient for connection to the EPG.

U.S. Patent No. 7,537,474 and 6,895,876, disclose a connector solution for an implantable pulse generator (IPG) utilizing a coiled spring inside a contact block. The ends of the spring are welded together yielding a torus shape through which the in-line lead is inserted. The spring coils cant to conform to the contact ring of an IPG lead, thus making electrical contact. Each coil which contacts both the lead and the block forms a separate redundant electrical contact.

However, current connectors that could be used for connecting the IPG contacts to a connector assembly as disclosed herein have a number of shortcomings. First, support for IPGs with up to, or more than, 24-26 contacts has not been supported. Furthermore, previous connectors typically used hand routed feed through (FT) wires to connect directly to the lead connection stack so no FT connection was required when lead frame designs were employed (e.g. MDT Restore) theFT pitch was approximately 50% larger and no stress relief was required due to a much larger available weld area than would be desirable. Furthermore, it would be useful to provide IPG devices with multiple lead ports that have contact stacks that are assembled as a single unit, and tested in a single fixture before final assembly of the IPG, to determine that all channels have electrical continuity to inserted electrode pin(s) that represents a connection end of a stimulation lead.

US 2008/0071320 A1 discloses a method for installing a connector assembly in a medical device according to the preamble of appended claim 1.

### SUMMARY OF THE INVENTION

The present invention is defined by the scope of appended claim 1. Further developments of the invention are in accordance with appended dependent claims 2 to 6.

The features and advantages described herein will become apparent to those skilled in the art upon reading the following description, with reference to the accompanying drawings, in which:
Figure 1 shows an example of a first arrangement of a lead frame for use with an example IPG;
Figure 2 shows another view of the lead frame of Figure 1;
Figure 3 shows an example of a contact assembly for use with the lead frame ofFigure 1;
Figure 4 shows a cross section of the example contact assembly of Fig. 3 flipped over;
Figure 5 shows the example contact assembly of Figure 3 with connecting leads of three example arrangements of lead frames installed;
Figure 5A shows a closeup of the example assembly of Figure 5;
Figure 6 shows the cross section of the contact assembly of Figure 4 with example electrode pins installed therein;
Figure 7 shows an example of the contact assembly of Fig. 3 connected to an IPG;
Figures 7A-7C shows vanous stages of assembly of an embodiment of contact assemblies connected to another example IPG using two lead frames;
Figure 8 shows a testing lead frame connected to the example IPG showing how the ends of leads of the lead frame may connect to corresponding IPG pins on the IPG;
Figure 9 shows a close up of how an example end of a lead of the example lead frame connects to an IPG pin of the example IPGs;
Figure 10 shows an example arrangement of IPG pins on the example IPGs;
Figure 11 shows the example of the second arrangement of a lead frame for use with the example IPG;
Figure 11A shows an embodiment of a first lead frame for use with the other example IPG;
Figure 12 shows the example of the third arrangement of a lead frame for use with the example IPG;
Figure 12A shows an embodiment of a second lead frame for use with the other example IPG;
Figure 13 is a block diagram showing example components of a pulse stimulation system using the example connector assembly including the example contact assembly and example lead frames; and
Figure 14 is a diagram illustrating an example medical application of the pulse stimulation system of Fig. 13.

### DETAILED DESCRIPTION OF THE EMBODIMENTS

Provided is a design for a welded feed through (FT) connector and its method of assembly and use that allows repeated flexing between a header and a hermetic enclosure of an active implantable medical device (e.g., an IPG) and minimizes stress to the electrical connection between the FT wire and the "lead frame" that leads to the contact stack for a stimulation lead. This is particularly important for welds made between dissimilar materials (e.g., Pt to MP35N or SS 316LVM) as these tend to have poorer mechanical attachment than those of similar materials (e.g., Pt to Pt).

The feed-through connection scheme described herein facilitates ease of assembly and long-term durability of a complete insert molded header to a hermetic enclosure containing an electronic stimulation circuit. The FT connector is especially designed for applications using laser welding of dissimilar materials such as platinum (Pt) IPG pins to MP35N or SS 316 LVM lead frame, but might also be useful for either similar material (e.g., Pt pins to Pt lead frame) or possibly resistance welding processes instead of laser welding.

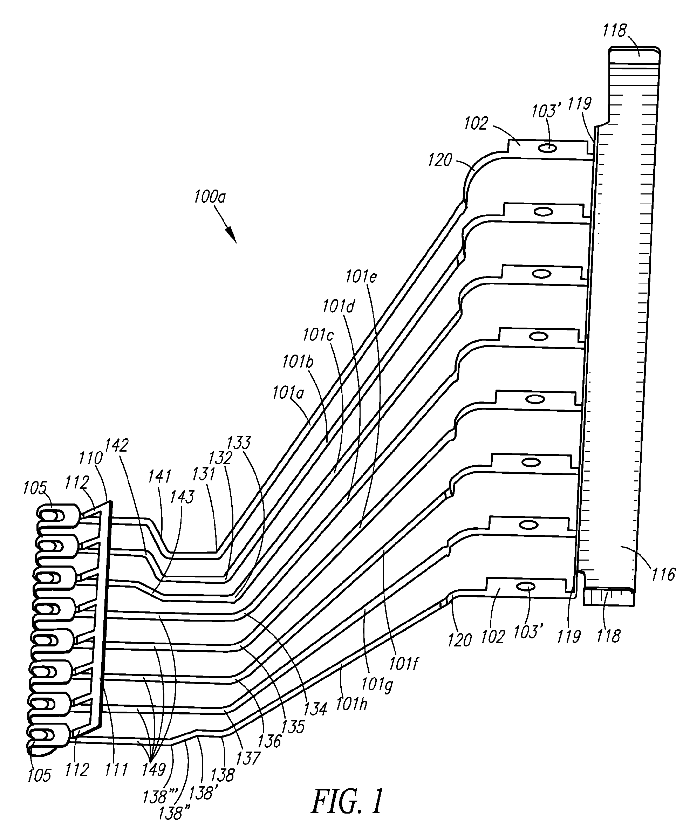

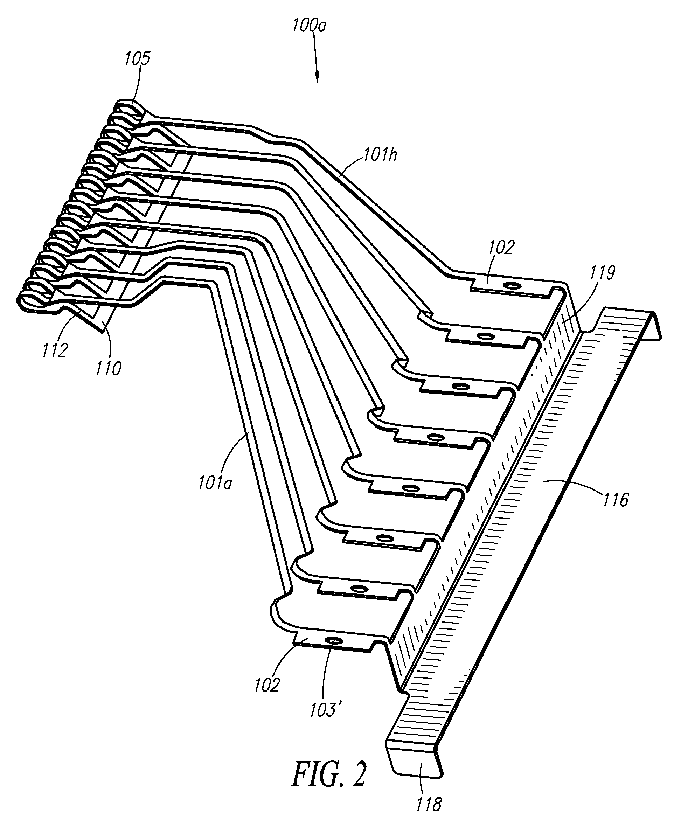

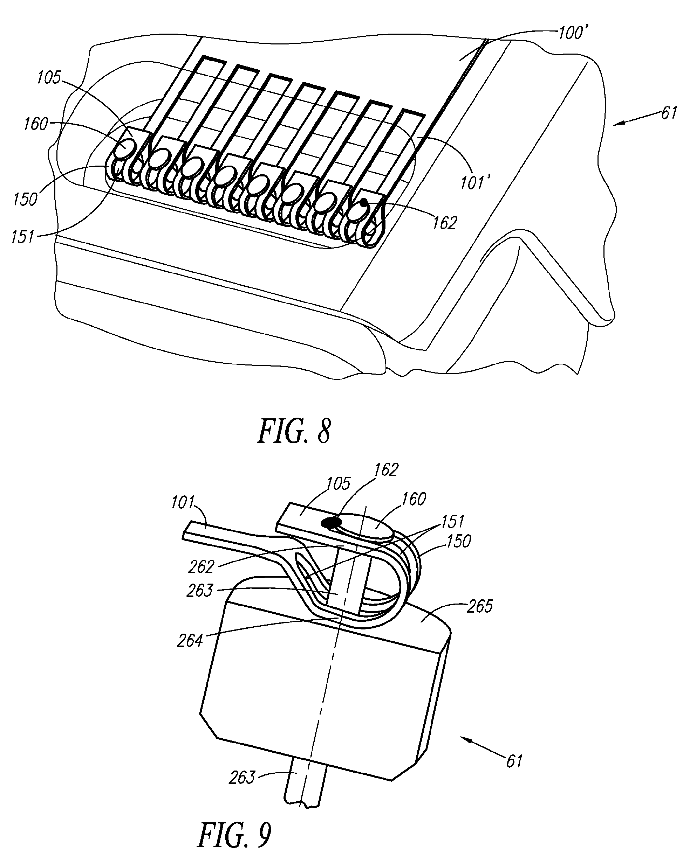

Figures 1 and 2 show different views of an example of a feed-through connector. The connector is arranged in a lead frame **100** after manufacture but before final use by connecting a plurality of leads **100** together by using a first temporary connecting structure **116** at one end of the lead frame **100** connected to the connector assembly connector lead end (CA lead end) **102** of each of the leads **101 at 119,** and a second temporary connecting structure **110** connected to the IPG connector end (IPG end) **105** of each of the leads **101 at 112.** Alternatively, as shown in Figure 8 for a testing lead frame **100',** the leads **101** can be connected together by using a example web, such as an insulating web. The web structure in Figure 8 is an example used for testing and proof of concept, whereas the actual product would utilize the structure like that shown in Figs. 1-2 and 7.

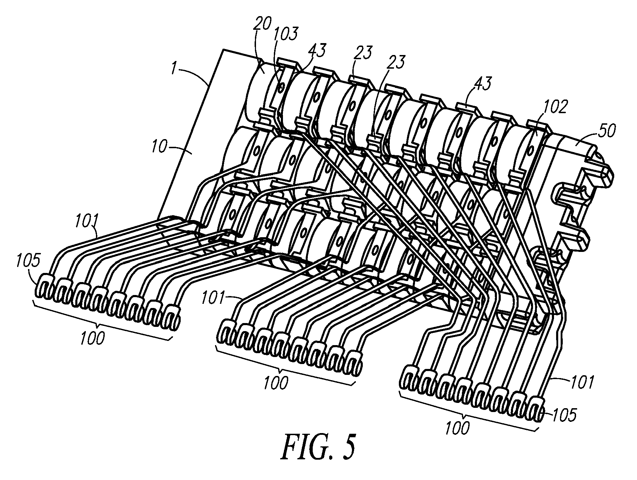

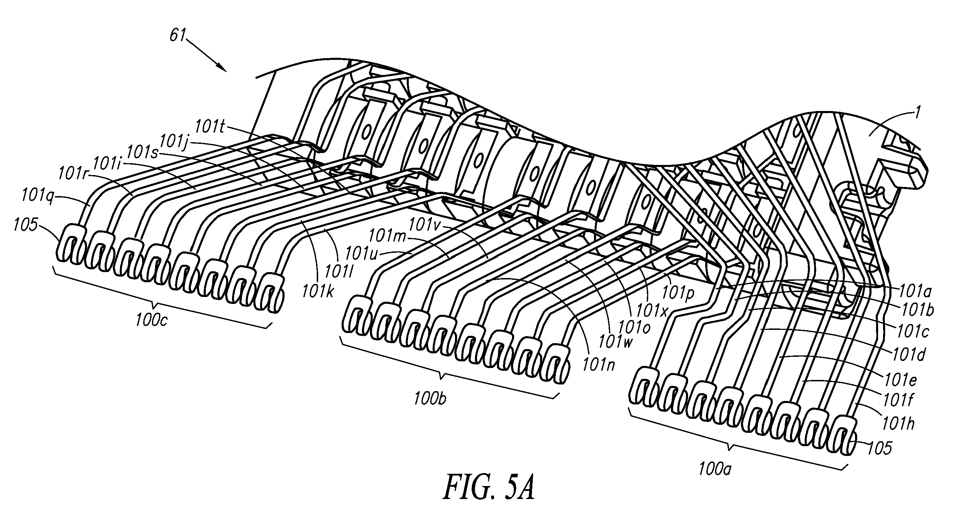

After installation, the temporary structures **116** and **110** can be removed, such as by breaking them off manually, for example. These structures would be made of the same materials as the lead frame itself, i.e., MP35N, Pt-Ir, or Stainless Steel, for example. The lead frame **100** has a plurality of leads **101** grouped together (as described above), such as into groups of 8 in the example embodiment. Typically, each lead frame **100** is formed into a shape to fit the contours of the IPG in which it will be installed. As shown in Figure 5, each one of the three lead frames **100a, 100b,** and **100c** are formed with different routings and bendings of the leads **101** for connecting to a connector assembly 1.

For example, in the example of Figs. 1 and 2, the leads **101** of lead frame **100** are formed into a particular shape for fitting as the rightmost lead frame **100a** of Figure 5. Each lead **101 (101a-101h)** has a CA lead end **102** formed into a wider portion (tab) with a weld hole **103',** the end **102** for connecting to a contact surface of a connector assembly by welding at weld hole **103'** (described below). TheCA lead ends **102** are all provided in a common plane for connecting to the connector assembly 1

The leads **101** are made narrower after the CA ends **102,** and have a curved portion **120** formed to route the leads to fit the IPG assembly. A relatively long run in the leads **101** is provided until a second curved portion 131-138 is provided in corresponding leads **101,** shown as **101a to 101h.** As shown, each of the curved portions **131-138** of the eight shown leads **101a-101h** has a different curvature, with the curve being much sharper the **131** on lead **101a** and gradually being less sharp (i.e., having a larger radius of curvature) in consecutive curves **132-136** (leads **101b-101g),** with lead **101g at 137** beginning to get sharper, and lead **101h** actually has a first curve **138,** a plateau portion **138'** a second curve portion **138",** and a third curve portion **138"'.**

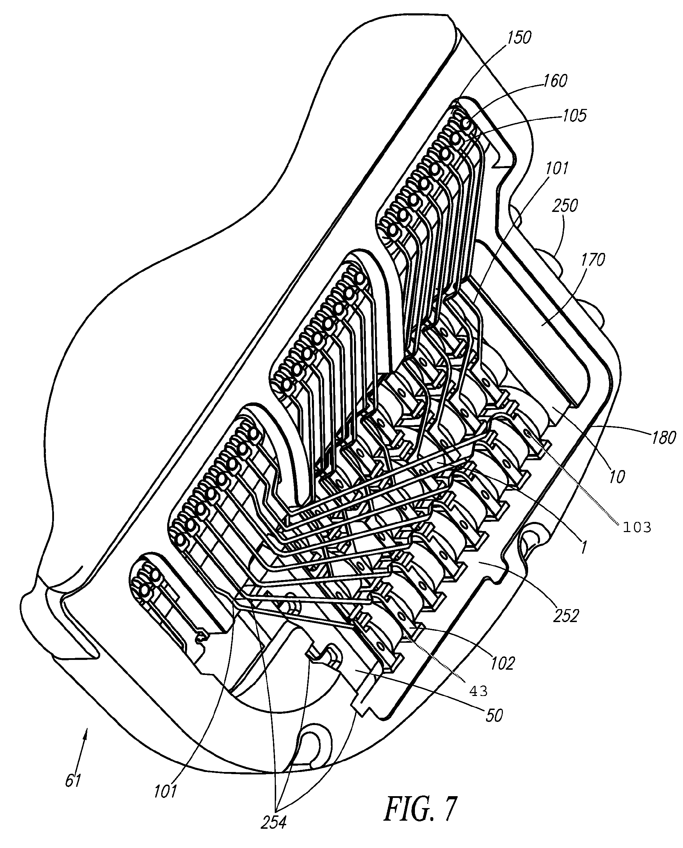

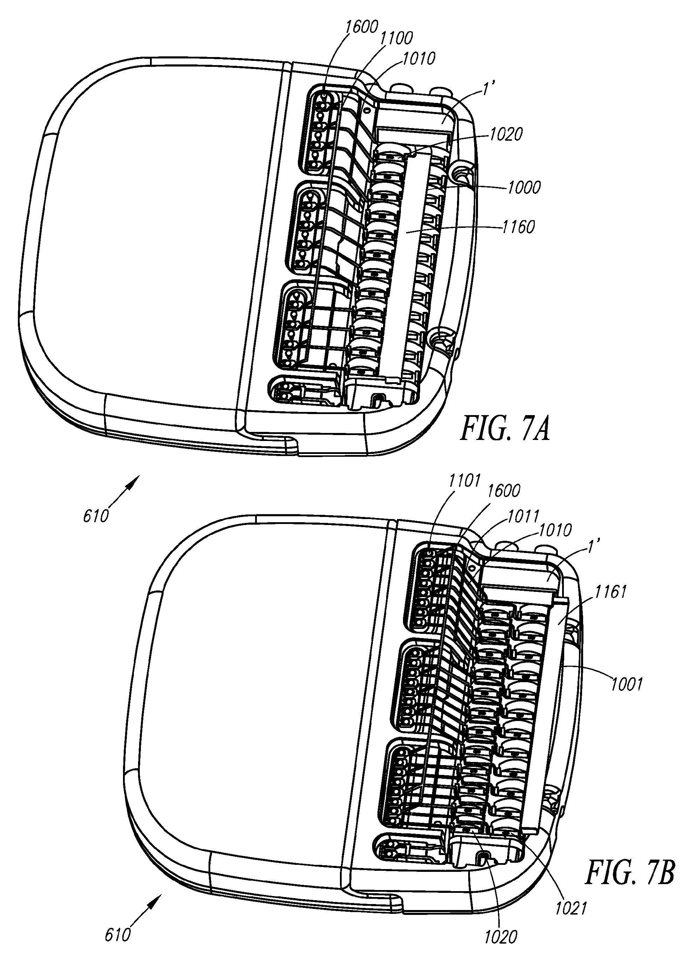

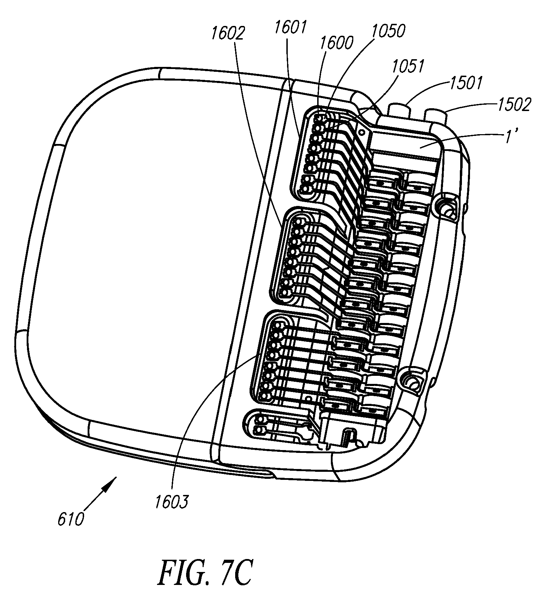

After these curves leads **101d-101h** have a relatively long flat portion 149, while three of the leads **101a-101c** have additional curved portions to step the leads up. Finally, the IPG ends **105** on each of the leads **101** are provided in a common plane, each for connecting to the IPG connector pins **160** of the IPG **61,** as shown in Figure 7.

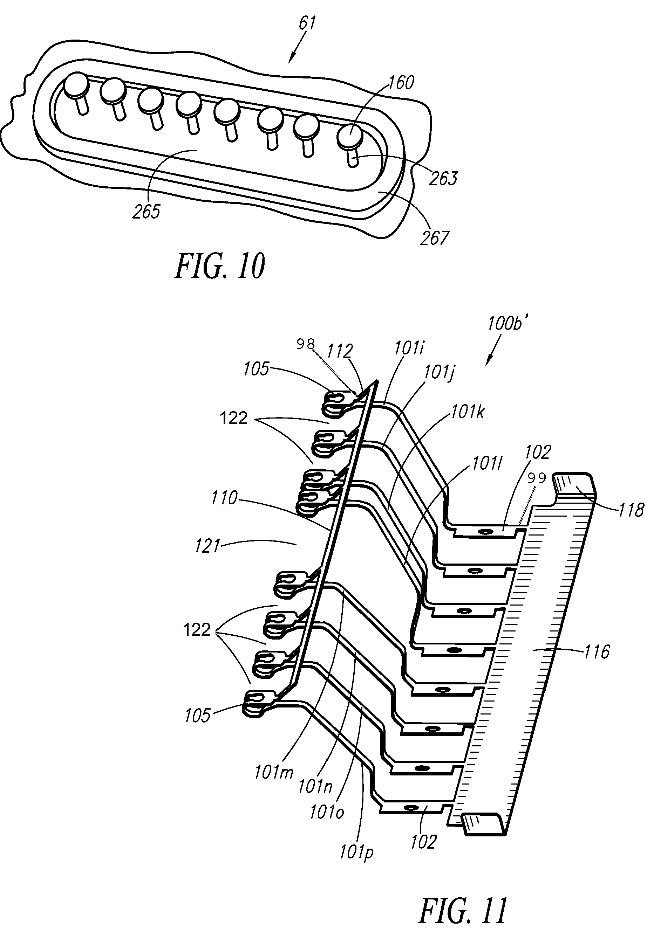

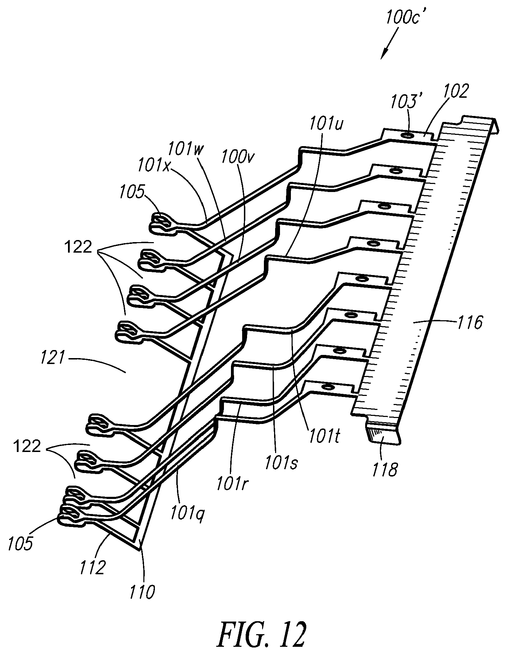

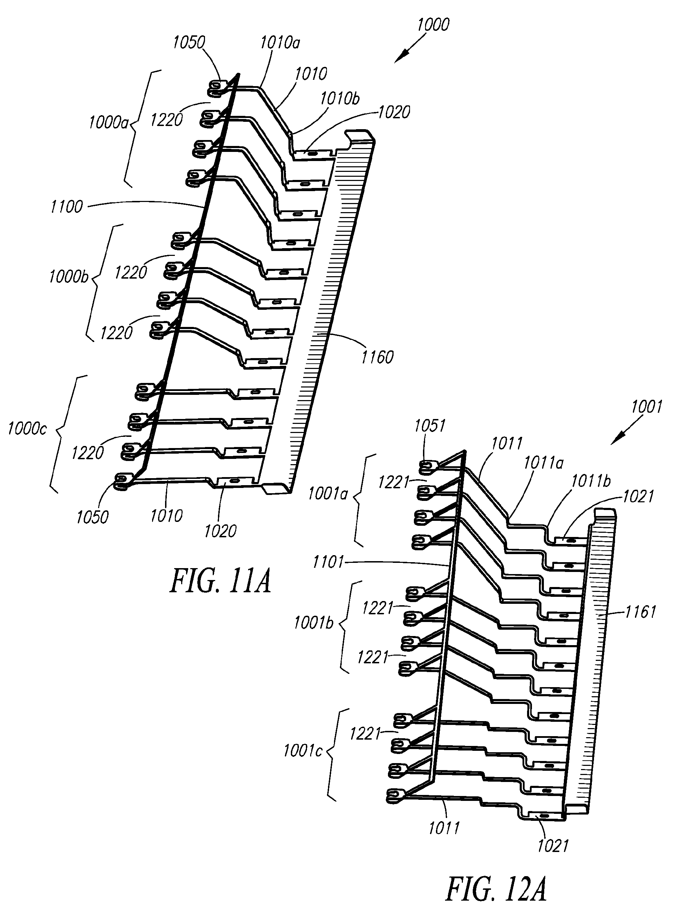

The other lead frames **100b'** and **100c'** are formed with leads routed differently in order to fit the contours within the IPG, as shown in Figures 11 and 12, as the specific routing is dependent on the design of the IPG and connector assembly, with the lead frame example routing described above merely being provided as an example to show the routing flexibility of the design. But as shown in Figure 7, all of the IPG ends **105** are coplanar and theCA ends **102** are coplanar in the example shown in that figure.

As shown in Figure 5A, for the Example embodiment using the example IPG, the lead frames **100b'** and **100c'** of Figures 11 and 12 are installed in a manner that interleaves the leads **101i-101p** oflead frame **100b'** (Figure 11) among some of the leads **101q-101x** of lead frame **100c'** (Figure 12) into groupings **100b** and **100c** of Figure 5A. Accordingly, the groupings **100b, 100c** of the leads **101** shown in Figure 5A do not correspond completely to the groupings of the individual leads of the lead frames **100b'** and **100c'** because of such interleaving, which "interleaves" some of the leads such that some of the leads of lead frame **100b'** are provided in grouping **100c** (i.e., leads **101i-1011)** whereas some of the leads of lead frame **100c'** are provided in grouping **100b** (i.e., leads **101u-101x)** in the manner illustrated in Figure 5A. The gaps **122** between the leads of the lead frames **100b'** and **100c'** thus allow for such interleaving of the leads, while the larger gaps **121** show where the leads transition from one grouping **100b** to the other grouping **100c.**

Figure 8 shows a testing lead frame **100'** with IPG lead ends **150** connected to an IPG **61** in the manner of the lead frame **100** (shown in Figure 7). As shown in Figure 9, the overall geometry and especially the U-shaped portion **150** of the IPG lead end **105** are configured to flex when the header is incidentally bent relative to the hermetic electronics enclosure of the IPG. This protects the welded portion of the connection from stress so that the mechanical integrity of the electrical connection is maintained. This benefit was clearly shown by a finite element analysis study of the proposed designs.

The weld **162** used for welding the IPG end **105** of the lead **101** to the IPG pin head **160** may or may not be provided on each of the ends **105,** as desired. The flexing of the end **105** (described in more detail below) may be sufficient to ensure electrical connectivity between the IPG pin **160** and the lead **101** without requiring welding all of the IPG ends **105,** but it is preferable to provide a **162** on each end **105** in order to ensure proper electrical connections and physical stability. If desired, multiple welds could be used on each end 10S to increase the reliability of the connection. In particular, two welds per end, provided opposite each other on the end **105** but in contact with the pin head **160,** would be useful for additional structural strength and electrical connectivity. Or one continuous weld over the pin head **160** could be utilized instead of multiple welds.

Further referring to Figure 9, it is shown that the IPG ends **105** have a flat portion **262** at the end of one branch of the U-shaped portion **150** for being placed against and electrically contacting the IPG pin head **160,** and a second flat portion **264** at the end of the other branch of the U-shaped portion **150** for being placed against the ceramic portion **265** of the IPG. These flat portions **262, 264,** in combination with the U-shaped portion **150,** cooperate to compress the flat portions **262, 264** against the respective head/ceramic surfaces to keep the IPG end in compression, and thus in place, and ensuring, in combination with the welds **162,** good electrical connectivity between the leads **101** and their respective IPG pins **263.** As shown, the split section **151** in the end **105** accepts the pin body portion of the pin **263.**

For the IPG **61** using the lead frame **100b'** of Figure 11, the assembly procedure proceeds as follows: The IPG ends **105** of lead frame **100b'** (figure 11) are positioned on the IPG pin heads **160** (figure 10) such that the U-shaped portion of IPG end **105** is engaged as in Figure 9. This is then accomplished for all IPG ends **105** oflead frame **100b'.** The lead frame **100b'** is then positioned so that all contact lead ends **102** lie directly upon conductive contact surfaces **43** of the contact blocks **40** (see Fig. 5) as depicted in Figure 7. After this is accomplished, a weld joint **162** (figure 9) is executed on each of the IPG ends **105** in order to mechanically and electrically join all the IPG ends **105** to the pin heads **160.** Next, welds are created at weld point **103** to join contact surface **43** to contact lead ends **102** (see Fig. 7). After welding lead frame **100b'** in the manner the first and second temporary connecting structures **116, 110** are dissected at separation points **98** and **99,** respectively, and removed, such as by manually bending and breaking them off, or by cutting them off with tools or lasers, for example. The similar process is repeated for the remaining lead frames, such as **100c'** (Fig. 12) and **100a,** to complete the lead frame attachment process for IPG **61.** Analogous operations are performed to assemble lead frames to IPG **610,** discussed below.

Figures 7A-7C show three views in various states of assembly of an embodiment using an IPG **610** for use with two electrode pins supporting 24 channels, with 12 channels per pin. In this case, two lead frames **1000** and **1001,** shown in figures 11A and 12A respectively, are comprised by the IPG **610.** This is in contrast to the IPG **61,** which utilizes three lead frames during assembly. Thus, the assembly ofiPG **610** is reduced by one lead frame insertion/assembly step.

The lead frame **1000** shown in Figure 11A is likewise comprised of a plurality of lead ends **1020,** an equal plurality of leads **1010** (some having bends such as **1010a** and **1010b,** for example), an equal plurality of IPG ends **1050** grouped by **1000a, 1000b,** and **1000c,** a first temporary connecting structure **1160,** and a second temporary connecting structure **1100.** The temporary connecting structures **1160, 1100** are used by an installer to insert the first lead frame **1000** into the IPG **610** as shown in Figure 7A (in a manner as discussed above regarding IPG **61).** Again, the leads **1010** have spaces **1220** between them that allow for additional leads to be interspersed in the manner shown in Figure 7B using the second lead from **1001** into the IPG **610.** The temporary connecting structures **1160/1100** are removed after the first lead frame.

Similarly, the second lead frame **1001** shown in Figure 12A is comprised of a plurality oflead ends **1021,** an equal plurality ofleads **1011** (some having bends such as **1011a** and **1011b,** for example), an equal plurality of IPG ends **1051** grouped by **1001a, 1001b,** and **1001c,** a first temporary connecting structure **1161,** and a second temporary connecting structure **1101.** The temporary connecting structures are used by an installer to insert the first lead frame **1001** into the IPG **610** as shown in Figure 7B (where lead frame **1000** is shown already installed) as discussed above. Again, the leads **1011** have spaces **1221** between them that allow for the leads **1010** oflead frame **1000** to be interspersed in the manner shown in Figure 7B.

Figure 7C then shows the final result IPG **610** with all temporary connecting structures having been removed. In other aspects this assembly is basically the same as discussed above with respect to IPG **61.** Note that in the example IPG **610,** each lead **1010** of the first lead frame **1000** is connected, via a lead end, to a contact block found in the same row of contact blocks that are associated with the electrodes of a first pin (entering at **1501**), whereas each lead **1011** of the second lead frame **1001** is connected to respective contact blocks of the other row of contact blocks that are associated with the electrodes of a second pin (entering at **1052**). But the IPG ends **1050** and **1051** of the respective lead frames are interspersed among each one of three sets **1601, 1602,** and **1603** of pin heads **1600** provided on the IPG **610** for each one of the stimulation channels.

For installation into an example IPG **610,** lead frame **1000** is first installed in a manner similar to that discussed for lead frame **100b',** above. In this case, the the lead ends **1050** oflead frame **1000** (figure 11A) are positioned on the IPG pin heads **1600** as shown in Figure 7A such that the U-shaped portion **1050** is engaged in a similar manner as shown in Figure 9. This is then accomplished for all lead ends **1050** of lead frame **1000** such that each lead end **1050** is installed on an alternate one of the pin heads **1600,** as shown partially installed by Figure 7A. Note that for this design, all lead frame lead ends 1020 are to be attached to all of the contact block contact surfaces that are closest to the IPG pins 1600 that are used to support a single lead bore **1501** (for accepting a single electrode pin, such as electrode pin **200**), in consecutive order. Weld joints on ends **1050** and **1020** are executed as discussed regarding lead frame **100b'.** Temporary connecting structures **1160, 1100** are then removed as discussed above regarding lead frame **100b'.**

Similarly, lead frame **1001** is then installed such that lead ends **1051** are engaged with those alternating pin heads **1050** that are not engaged with lead frame **1000.** Note that for this second lead frame **1001,** all lead frame lead ends **1021** are to be attached to all of the contact block contact surfaces that are furthest from the IPG pins **1600** supporting another single lead bore **1502** (for accepting another electrode pin), again in consecutive order. Again, the proper weld joints are executed on ends **1051** and **1021,** and the temporary connecting structures **1101** and **1161** are removed, completing the installation of the lead frames on IPG **610.**

One advantage to the lead frame design discussed above for IPG 610, in addition to requiring the use and installation of one fewer lead frame, is that because each lead frame is installed on the pin heads in a regular manner (i.e., on alternating pin heads), there is little confusion as to which connections are required. Also, because all lead frame ends of a given lead frame attach to consecutive adjacent contact blocks, this also leads to a lesser chance of confusion on installation, making erroneous installations less likely.

Some of the advantages of these designs disclosed herein are:
1. Protects the welded joint **162** from stress due to flexing:
2. The assembly is self fixturing because of two features: Referring to figures 7-9, the split section **151** in the U-shaped portion 150 of the IPG Lead ends **105** of the individual leads 101 of the lead frame **100/100'** causes the individual connection features on the lead frame to self align with its respective IPG pin **263.** When the U-shaped portion **150** of the IPG lead end **105** is dimensioned such that the parallel sides of the U are slightly more separated than the high dimensional limit of the space between the IPG ceramic portion **265** and the underside of the IPG pin head **160** the two elements are then positioned so as to provide intimate contact for the weld zone **162,** a desirable condition for a successful weld.
3. The lead frame is further configured so that when the lead frame experiences distortion due to header bending, it remains clear of the FT flange **267** (see Figure 10, which shows a partial view of an example arrangement of the IPG pins **263** on an IPG **61**). This protects against the possibility of the leads **101** of the lead frame **100** making an electrical short to theFT flange **267.**
4. The preceding benefits can all be accomplished at a compact spacing of 1.27 cm (.05") between the IPG pins **263,** allowing for an IPG device with a higher number of contacts in a smaller overall device configuration that would otherwise be possible.

The lead frame can be utilized with a stack connector assembly that allows IPG devices with multiple lead ports to have contact stacks that are assembled as a single unit and tested in a single fixture before assembly to determine that all channels have electrical continuity to an inserted electrode pin that represents the connection end of a stimulation lead or plurality of such leads. Figure 5 (described in more detail below) shows how the lead frame, with removable parts removed, would be connected to such a connector assembly **1.**

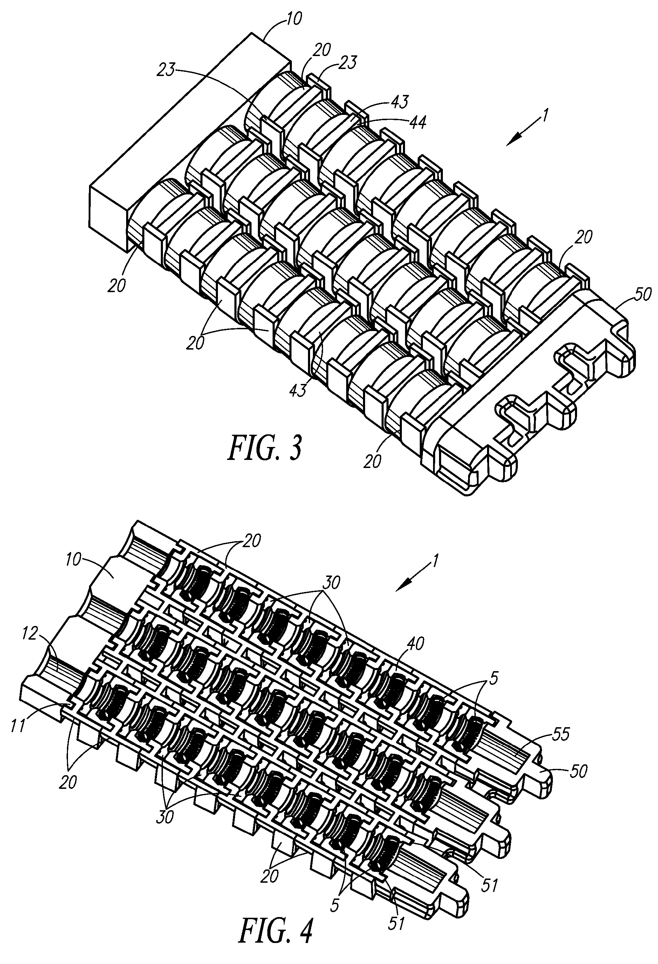

Figure 3 shows an example schematic of an example contact assembly that can be used with the lead frame discussed above. Figure 4 shows a flipped cross section of the assembly of Figure 3. The example contact assembly is comprised of a setscrew block **10** and an end cap **50** covering opposite ends of the contact assembly. A plurality of thermoplastic stacker components **20** are shown provided between the setscrew block **10** and the end cap **50.** For the example there is one stacker component per "row". Each of the thermoplastic stacker components is associated with a set of conductive contact blocks **40** (three per row are shown in the example representing three "columns"), a set of corresponding seals **30** (3 per row in the example), and a set of contacts, such as springs **5** (again, 3 per row in the example). For this example shown in the figures, there are eight sets of stacker components (i.e., forming 8 "rows") with each stacker component (and thus each row) associated with a set of three contact block/seaVspring groupings (i.e., three "columns"). Of course, alternative examples could utilize alternative numbers of contact blocks (i.e., different numbers of rows), and each contact block might be associated with a different number of contact block/seaVspring groupings (i.e., different numbers of columns), such as using a single grouping, or two, four, five, or more groupings, depending on the desired implementation. Alternatively, the stacker components could be comprised of separate sub-components each associated with one of the contact blocks (hence for the example, there would be three sub-components).

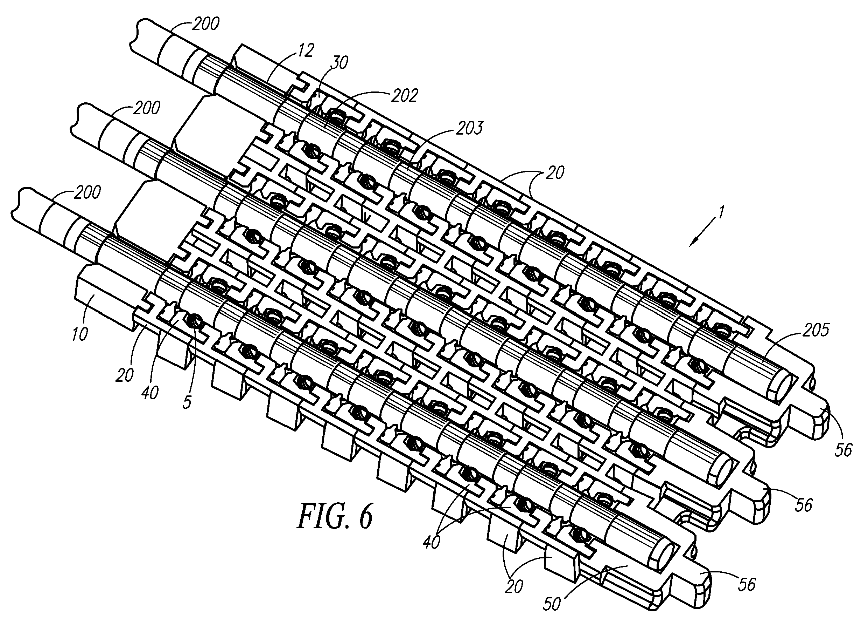

The stacker component **20** has, for example, three open central portions including holes (bores), for receiving correspond electrode pins **200** as described below (see Fig. 6). For any given stacker component, each open central portion is adapted for receiving a corresponding seal **30.** Each seal **30** has a hole 33 formed in its center that is aligned with an associated hole of the stacker component for receiving the corresponding electrode pin **200.**

Each stacker component is adapted to receive, on one side, either a part of a contact block **40** or a part of a setscrew block **10,** while another side is adapted to receive a part of another contact block **40** between two block tabs 23 of the stacker component **20.**

Each contact block **40** has a conductive contact surface **43** on contact tab **44** that, when paired with the second side of an associated stacker component **40,** is exposed between the associated pair of block tabs **23** of the stacker component, the conductive contact surface **43** being exposed for electrically connecting to a contact lead **102** (see Fig. 7). Each contact block **40** also has an interior hole and a hollowed out interior portion with a groove for holding a corresponding spring **5.** Each spring **5** is formed in a ring (donut) shape of conductive material with a void in its center (for receiving the associated electrode pin **200)** and is in electrical contact with its corresponding contact block to ensure electrically conductivity.

The springs **5** are, in the example torsion springs formed into a ring (a toroid/"donut" shape) having a space or hole in the center for receiving the corresponding electrode pin **200** (see Fig. 6). When the electrode pins **200** are inserted therethrough, the springs flex (cant) against and in electrical contact with a corresponding conductive surface portion, such as conductive ring **202** on the surface of the electrode pin **200** to make electrical contact with the conductive ring **202,** as illustrated in Fig 6. Alternative means of providing electrical contact between the electrode pin conductive rings 202 and the contact blocks could also be provided, such as by using metal tabs or different spring mechanisms, if desired, or integrating a contact structure directly in the contact block itself.

In most situations, each contact block is received by (mates with) a first side of a following stacker component **20** which acts to "cap" the components of a previous row assembled in the stacker component **20** and help hold them in place. The contact tab **44** fits between the block tabs **23,** with the block tabs **23** extending beyond corresponding ends of the contact tab **44** and exposing a flat outer contact surface **43.** If the contact block **40** is part of the last row of the device **1,** the second cylindrical portion **42** is instead received by (mates with) the end cap **50.**

Hence, for the example each stacker component **20** is associated with a plurality (three each in the example embodiment) of seals **30,** three contact blocks **40,** and three springs **5,** to create a row (layer).

Figure 5 shows the contact assembly of the example connected to three sets (groups) of lead frames **100,** each having 8 leads **101,** with each lead **101** having an end **102** adapted for being attached to the exposed outer contact surface **43** of a corresponding contact tab **44** (see, e.g., Figs. 3-4) and having a weld point **103.** The leads **101** are conveniently routed in an organized manner around the tabs and other components of the stacker components to avoid shorting any of the leads together, such that each of the lead frames have leads that are routed differently from each other (as shown by the examples of Figures 1-2, versus the differently routed lead fames shown in Figures 10 and 11). Each of the leads **101** has a connector **105** at the other one end for connecting to a corresponding electrical connection point **160** on the IPG (as shown in Figure 7). Thus, a conductive path is provided from one point **160** on the IPG, to the lead connector **105** connected to the point **160,** down the associated lead **101** to the other end **102** to the corresponding contact block **40** to which the end **102** is connected, through the contact block to the associated spring **5** inserted therein, and on to the corresponding conductive ring **202,** in contact with the spring **5,** of the electrode pin **200** inserted through that contact block **40,** and ultimately to an electrode, such as might be implanted near the spine of a patient for providing pulse therapy.

As shown in example of Figure 6, three bores (columns) are defined through each of the eight rows of example contact assembly **1,** with each of the bores/columns for receiving the corresponding one of the electrode pins **200** (thereby supporting three pins of eight conductors each). The pins are comprised of conductive rings 202 (each corresponding to one of the conductors of the electrode pin) and insulating portions 203. Because each pin supports a plurality of conductors, each pin can support a like plurality of electrodes for the desired medical therapy. Each of the bores is defined by the appropriate axial alignment of one of the holes **12** provided through the setscrew block **10,** the hole (33) of one of the seals **(30)** in each row (inserted in its corresponding contact holder 25), and the hole (center) of one of the springs **5** (that are inserted in their corresponding of the contact block **40)** in each row, and finally to one of the bores **55** of the end cap **50,** in a manner sufficient to ensure that the electrode pins are adequately held in place and provide the appropriate electrical contacts to the associated contact blocks.

The setscrew block **10** is preferably comprised of titanium, although it could be comprised of any strong biocompatible metal such as stainless steel, nickel alloys, etc. The block can be manufactured using a machining process, or a metal injection molding (MIM) process, for example. The setscrew block holds setscrews (not shown) that tighten on the electrode pins **200** setscrew rings and prevent the leads from moving out of alignment with the contacts and seals of the contact assembly. The setscrew block **10** has a set of three screws (not shown) that are used to set (fix) the electrode pins **200** in place, once inserted, although other means of fixing the pins in place could be utilized, or the electrode pins may be kept in place solely by friction contact with the seals and springs through which they pass, or by some other mechanism.

Each of the stacker components **20** is preferably comprised of a polymer such as Polysulfone, but it could be any biocompatible polymer or other composition of similar capability. The components **20** can be manufactured by using Injection molding, or a machining process suitable for its composition and size. The stacker components **20** hold the seals **30** and contact blocks **40** in alignment (axially and radially), control seal compression, and act as a precision spacer to maintain contact to contact pitch. In the example embodiment, the stack pitch is about 0.254 cm (.100") nominal and accepts an electrode pin of about 0.14 cm (.055") nominal diameter. This concept will work down to around 0.202 cm (.80") pitch and pretty much any diameter (limited by how small the toroidal springs can be wound). The tolerance in the stacker contributes to the overall stack tolerance, likewise each of the seals is can be compressed as a separate assembly, so compression is controlled by the tolerances in one contact block and one seal not by the stack in its entirety.

Each of the seals **30** is preferably comprised of an elastic material such as silicone, or another elastomeric biocompatible polymer, and can be manufactured by molding, for example. Alternatively, the seals could be molded directly onto the stackers so they would form a single piece. The seals align with nonconductive segments **203** between the contact points on the electrode pin **200** and conform to the electrode pin surface so that even if flooded with conductive liquid in the lead bore, adjacent contacts have a sufficiently high enough impedance (i.e. 50k Ohms) between them that they cannot effectively communicate electrically.

Each of the contact blocks **40** is preferably comprised of an MP35N alloy (a commercially available nonmagnetic, nickel-cobalt-chromium-molybdenum alloy that has a unique combination of properties), although any conductive biocompatible metal or alloy could be used. The contact blocks **40** can be manufactured by using a metal injection molding (MIM) process, or machined using known machining methods. The contact blocks are used to make electrical contact with the springs **5,** transfer electrical signals from the electrode pins **200** to the leads **101,** form a weld surface for the leads **101,** and compress the seals **30** (in conjunction with the stackers components **20).**

The springs **5** are comprised of a small diameter (e.g., 0.0089 cm (.0035") or less) coiled Pt-Ir wire joined into a continuous toroidal shaped helix. The assembly can be made compatible with, and thus utilize, springs such as those disclosed in U.S. Pat. No. 6,749,358 and 7,070,455, and U.S. Pat. App. Pub. No. 2008/0246231, incorporated herein by reference.

The end cap **50** is preferably comprised of the same or similar material discussed for the stacker components **20.** Alternatively, the end cap **50** could be comprised of a biocompatible metal with the inclusion of additional seals to ensuring sealing, in particular where a conductive end cap might be desirable. The end cap forms the end of the pin bores and the depth of the holes **55** providing in the end caps (for receiving the end 205 of the electrode pins **200)** registers the location where the electrode pins align with the rest of the stack.

The contact assembly **1** can be assembled on assembly pins, such as the electrode pins **200** or by using other pins of the appropriate size for aid in arranging the assembly components. For the example shown in the figures, one electrode pin is used through each one of the three bores. The pins help to maintain alignment in the stack and make the components and the stack easier to handle. For the example the assembly is accomplished manually by hand, but could be automated where mass production is contemplated to cover the cost of the machine and robotics.

The assembled contact assembly with IPG pins therein is placed into a shell or housing **180.** The shell has a feature (including the slots **254** of Figure 7) that interlocks with the end cap tabs **56** on one side, including a vertical wall that forms a hard stop for the end cap **50.** The other side of the shell **180** has an elastomeric piece **170** through which the bores continue through the cylinders **250.** The assembly pins are inserted through the cylinders **250** of the elastomeric piece **170** first, then the curved and angled surfaces of the shell **180** and end cap **50** allow the rest of the stack to be pushed into place. Now the connector stack is trapped in alignment between the hard stop at the end cap **50** and the elastomeric piece **170** which serves as a spring to hold the stack in compression. The assembly pins can then be removed. The shell can then be attached to the IPG (or possibly was pre-attached). Then the lead frames **100** are attached to the IPG and the contact blocks, with the leads **101** being welded or soldered to the contact tab 43 at weld/solder point **103** (see Fig. 5). Then the shell is filled with a potting material **252,** such as silicone, for example. The potting material **252** surrounds the contact assembly and each of the leads and the IPG connection points to insulate the contact assembly electrically and physically hold the components in alignment to one another and binds the assembly together.

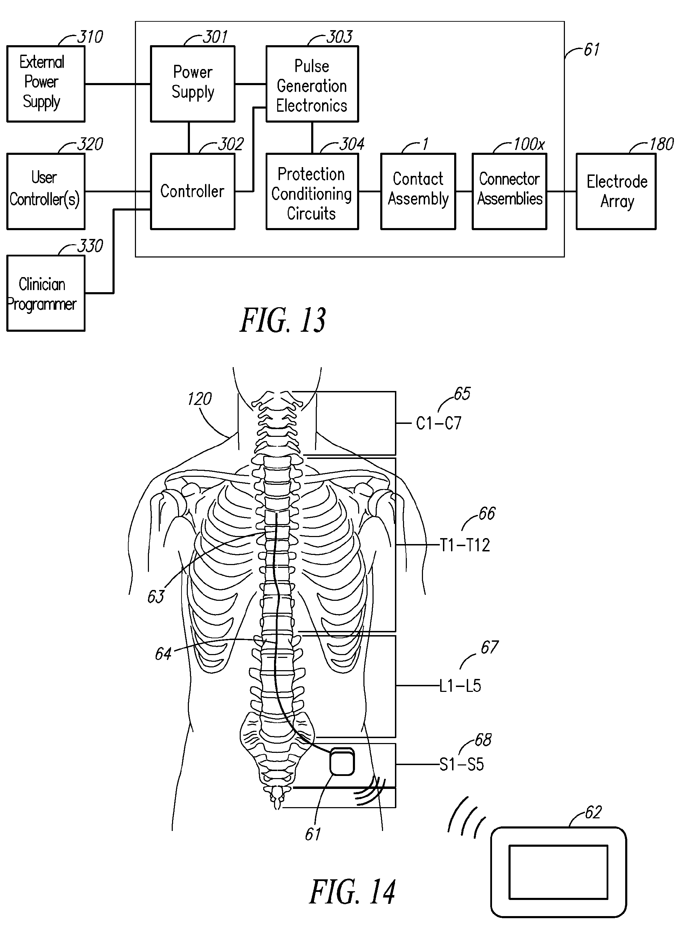

Figure 13 provides a block diagram of an example system including an IPG **61** that could utilized the contact assembly **1.** The IPG **61** can be comprised of an internal power supply **301** (that may include a rechargeable battery), a controller **302,** pulse generation electronics **303,** protection/conditioning circuits **304,** and the contact assembly **1** for connecting to an electrode array **180.** The IPG **61** can be supported by an external power supply **310** (such as for charging the battery of the internal power supply **301),** and a clinician programmer **330** and a user controller **320.**

Figure 14 shows an example application of the stimulator system for providing spinal stimulation. In that figure, the IPG **61** is shown implanted in a patient. Also shown is the human spine comprising the C1-C7 cervical vertebrae **65,** the T1-T12 thoracic vertebrae **66,** the L1-L5 lumbar vertebrae 67, and the S1-S6 sacral vertebrae **68.** Electrodes **63** are shown disposed at the distal end of the spine and are positioned near the thoracic vertebrae **66.** The Electrodes **63** are attached to the IPG **61** via electrode leads **64.**

The leads and electrodes may be positioned anywhere along the spine to deliver the intended therapeutic effects of spinal cord electrical stimulation in the desired region of the spine. The distal end of the lead with its accompanying electrodes may be located along the epidural space and adjacent a desired portion of the spinal cord using well-established and known techniques for implanting and positioning SCS leads and electrodes, and the IPG **61** may be programmed using a clinician or other type of programmer **62** (such as a patient controller), as desired (and further described above). The electrode leads **64** can be connected to the IPG via a contact assembly as described in this application.

Many other examples can be provided through various combinations of the above described features. Although the embodiments described hereinabove use specific examples and alternatives, it will be understood by those skilled in the art that various modifications can be made without departing from the intended scope of the appended claims.

## Claims

1. A method for installing a connector assembly in a medical device (610) including a plurality of conducting pins (1600) for connecting said medical device (610) to a connector stack including a plurality of contact blocks, said method comprising:
providing a first lead frame (1000) comprising a first plurality of conductive leads (1010), wherein the first plurality of conductive leads (1010) includes all conductive leads (1010) of the first lead frame (1000) and
wherein each of the conductive leads (1010) has a first end (1050) and a second end (1020);
fixedly connecting said first end (1050) of each one of said first plurality of leads (1010) to corresponding ones of said plurality of conducting pins;
fixedly connecting said second end (1020) of each one of said first plurality of leads (1010) to corresponding ones of said contact blocks;
wherein said first lead frame (1000) has at least one temporary connecting structure (1100, 1160) connecting said first plurality of conductive leads (1010) together and that is removed from the first plurality of conductive leads (1010) as said first lead frame (1000) is installed in said medical device (610);
**characterized by**:
fixedly connecting said first end (1050) of each one of said first plurality of leads (1010) to corresponding ones of said plurality of conducting pins (1600) such that only alternate ones of said plurality of conducting pins (1600) are connected to corresponding first ends (1050) of said conductive leads (1010) of the first plurality of conductive leads (1010) and such that all conductive leads (1010) of the first plurality of conductive leads (1010) are connected to conducting pins (1600);
providing a second lead frame (1001) including a second plurality of conductive leads (1011) each having a first end (1051) and a second end (1021);
fixedly connecting said first end (1051) of each one of said leads of said second lead frame (1001) to corresponding ones of said conducting pins (1600) that are not connected to any of said first plurality of leads (1010); and
fixedly connecting said second end (1021) of each one of said second plurality of leads (1011) to corresponding ones of said contact blocks different than the contact blocks connected to any of said first plurality of leads (1010),
wherein
the first plurality of conductive leads (1010) and the second plurality of conductive leads (1011) are interleaved together.

2. The method of claim 1, wherein said step of fixedly connecting comprises the step of welding to accomplish said fixedly connecting.

3. The method of claim 1 or 2, wherein the at least one temporary connecting structure comprises a temporary connecting structure connecting said first ends (1050) or said second ends (1021) of said first plurality of conductive leads (1010) together, wherein said temporary connecting structure (1100, 1160) is removed as said lead frame (1000) is installed in said medical device (610).

4. The method of claim 3,
further comprising another temporary connecting structure (1100, 1160) connecting the other of said first ends (1050) or said second ends (1021) of said first plurality of conductive leads (1010) together, wherein said temporary connecting structures (1100, 1160) are removed as said lead frame (1000) is installed in said medical device (610).

5. The method of claim 1,
wherein said contact blocks to which said second end (1020) of said first plurality of conductive leads (1010) that is fixedly connected are provided on a single bore of said medical device (610) for supporting an electrode pin; and/or
wherein said contact blocks to which said second end of said first plurality of conductive leads (1010) that is fixedly connected are distributed among more than one bore of said medical device (610) for supporting a plurality of electrode pins.

6. The method of claim 1,
wherein said first lead frame (1000) includes a first temporary connecting structure (1100) connecting the first ends (1050) of said first plurality of conductive leads (1010) together, and a second temporary connecting structure (1160) connecting the second ends (1020) of said first plurality of conductive leads (1010) together, both said first connecting structure (1100) and said second connecting structure (1160) being removed during assembly, and
wherein said second lead frame (1001) includes a third temporary connecting structure (1101) connecting the first ends (1051) of said second plurality of conductive leads (1011) together, and a fourth temporary connecting structure (1161) connecting the second ends (1021) of said second plurality of conductive leads (1011) together, both said third connecting structure (1101) and said fourth connecting structure (1161) being removed during assembly.

## Patentansprüche

1. Verfahren zur Anbringung einer Steckverbinderanordnung in einer medizinischen Vorrichtung (610), welche eine Vielzahl von Anschlussstiften (1600) zum Verbinden der medizinischen Vorrichtung (610) mit einem Steckverbinderstapel beinhaltet, der eine Vielzahl von Kontaktblöcken beinhaltet, wobei das Verfahren umfasst:
Bereitstellen eines ersten Leiterrahmens (1000), der eine erste Vielzahl leitfähiger Anschlussdrähte (1010) umfasst, wobei die erste Vielzahl leitfähiger Anschlussdrähte (1010) alle leitfähigen Anschlussdrähte (1010) des ersten Leiterrahmens (1000) beinhaltet und wobei jeder der leitfähigen Anschlussdrähte (1010) ein erstes Ende (1050) und ein zweites Ende (1020) aufweist;
fest Verbinden des ersten Endes (1050) jedes der ersten Vielzahl von Anschlussdrähten (1010) mit entsprechenden der Vielzahl von Anschlussstiften;
fest Verbinden des zweiten Endes (1020) jedes der ersten Vielzahl von Anschlussdrähten (1010) mit entsprechenden der Kontaktblöcke;
wobei der erste Leiterrahmen (1000) mindestens eine vorläufige Verbindungsstruktur (1100, 1160) aufweist, welche die erste Vielzahl leitfähiger Anschlussdrähte (1010) miteinander verbindet und welche von der ersten Vielzahl leitfähiger Anschlussdrähte (1010) entfernt wird, wenn der erste Leiterrahmen (1000) in der medizinischen Vorrichtung (610) angebracht ist;
**gekennzeichnet durch**:
fest Verbinden des ersten Endes (1050) jedes der ersten Vielzahl von Anschlussdrähten (1010) mit entsprechenden der Vielzahl von Anschlussstiften (1600), sodass nur abwechselnde der Vielzahl von Anschlussstiften (1600) mit entsprechenden ersten Enden (1050) der leitfähigen Anschlussdrähte (1010) der ersten Vielzahl von Anschlussdrähten (1010) verbunden sind, und sodass alle leitfähigen Anschlussdrähte (1010) der ersten Vielzahl leitfähiger Anschlussdrähte (1010) mit Anschlussstiften (1600) verbunden sind;
Bereitstellen eines zweiten Leiterrahmens (1001), der eine zweite Vielzahl leitfähiger Anschlussdrähte (1011) beinhaltet, die jeder ein erstes Ende (1051) und ein zweites Ende (1021) aufweisen;
fest Verbinden des ersten Endes (1051) jedes der Anschlussdrähte des zweiten Leiterrahmens (1001) mit entsprechenden der Anschlussstifte (1600), die nicht mit einem der ersten Vielzahl von Anschlussdrähten (1010) verbunden sind; und
fest Verbinden des zweiten Endes (1021) jedes der zweiten Vielzahl von Anschlussdrähten (1011) mit entsprechenden der Kontaktblöcke, die von den mit einem der ersten Vielzahl von Anschlussdrähten (1010) verbundenen Kontaktblöcken verschieden sind,
wobei
die erste Vielzahl leitfähiger Anschlussdrähte (1010) und die zweite Vielzahl leitfähiger Anschlussdrähte (1011) ineinander verschachtelt sind.

2. Verfahren nach Anspruch 1, wobei der Schritt des fest Verbindens den Schritt des Schweißens umfasst, um dieses feste Verbinden zu bewerkstelligen.

3. Verfahren nach Anspruch 1 oder 2, wobei die mindestens eine vorläufige Verbindungsstruktur eine vorläufige Verbindungsstruktur umfasst, welche die ersten Enden (1050) oder zweiten Enden (1021) der ersten Vielzahl leitfähiger Anschlussdrähte (1010) miteinander verbindet,
wobei die vorläufige Verbindungsstruktur (1100, 1160) entfernt wird, wenn der Leiterrahmen (1000) in der medizinischen Vorrichtung (610) angebracht ist.

4. Verfahren nach Anspruch 3,
weiter eine andere vorläufige Verbindungsstruktur (1100, 1160) umfassend, welche die anderen der ersten Enden (1050) oder zweiten Enden (1021) der ersten Vielzahl leitfähiger Anschlussdrähte (1010) miteinander verbindet, wobei die vorläufigen Verbindungsstrukturen (1100, 1160) entfernt werden, wenn der Leiterrahmen (1000) in der medizinischen Vorrichtung (610) installiert ist.

5. Verfahren nach Anspruch 1,
wobei die Kontaktblöcke, womit das zweite Ende (1020) der ersten Vielzahl leitfähiger Anschlussdrähte (1010) fest verbunden ist, an einer einzigen Bohrung der medizinischen Vorrichtung (610) vorgesehen sind, um eine Stiftelektrode zu tragen; und/oder
wobei die Kontaktblöcke, womit das zweite Ende (1020) der ersten Vielzahl leitfähiger Anschlussdrähte (1010) fest verbunden ist, über mehr als eine Bohrung der medizinischen Vorrichtung (610) verteilt sind, um eine Vielzahl von Stiftelektroden zu tragen.

6. Verfahren nach Anspruch 1,
wobei der erste Leiterrahmen (1000) eine erste vorläufige Verbindungsstruktur (1000), welche die ersten Enden (1050) der ersten Vielzahl leitfähiger Anschlussdrähte (1010) miteinander verbindet, und eine zweite vorläufige Verbindungsstruktur (1160), welche die zweiten Enden (1020) der ersten Vielzahl leitfähiger Anschlussdrähte (1010) miteinander verbindet, beinhaltet, wobei sowohl die erste Verbindungsstruktur (1100) als auch die zweite Verbindungsstruktur (1160) während des Zusammenbaus entfernt werden, und
wobei der zweite Leiterrahmen (1001) eine dritte vorläufige Verbindungsstruktur (1101), welche die ersten Enden (1051) der zweiten Vielzahl leitfähiger Anschlussdrähte (1011) miteinander verbindet, und eine vierte vorläufige Verbindungsstruktur (1161), welche die zweiten Enden (1021) der zweiten Vielzahl leitfähiger Anschlussdrähte (1011) miteinander verbindet, beinhaltet, wobei sowohl die dritte Verbindungsstruktur (1101) als auch die vierte Verbindungsstruktur (1161) während des Zusammenbaus entfernt werden.

## Revendications

1. Procédé pour le montage d'un assemblage de connecteurs dans un dispositif médical (610) englobant plusieurs broches conductrices (1600) pour raccorder ledit dispositif médical (610) à un empilage de raccords englobant plusieurs blocs de contact, ledit procédé comprenant le fait de :
procurer un premier cadre de montage (1000) comprenant une première série de fils conducteurs (1010), ladite première série de fils conducteurs (1010) englobant tous les fils conducteurs (1010) du premier cadre de montage (1000) et chacun des fils conducteurs (1010) possède une première extrémité (1050) et une deuxième extrémité (1020) ;
raccorder à demeure ladite première extrémité (1050) de chacun des fils conducteurs de ladite première série de fils conducteurs (1010) à des broches conductrices correspondantes parmi lesdites plusieurs broches conductrices ;
raccorder à demeure ladite deuxième extrémité (1020) de chacun des fils conducteurs de ladite première série de fils conducteurs (1010) à des blocs de contact correspondants parmi lesdits blocs de contact ;
dans lequel ledit premier cadre de montage (1000) possède au moins une structure de raccordement temporaire (1100, 1160) qui raccorde les uns aux autres les fils conducteurs de ladite première série de fils conducteurs (1010) et qui est retirée de ladite première série de fils conducteurs (1010) lorsque ledit premier cadre de montage (1000) est monté dans ledit dispositif médical (610) ;
**caractérisé par** le fait de :
raccorder à demeure ladite première extrémité (1050) de chaque fil conducteur de ladite première série de fils conducteurs (1010) à des broches conductrices correspondantes parmi lesdites plusieurs broches conductrices (1600) d'une manière telle que seulement une broche sur deux parmi lesdites plusieurs broches conductrices (1600) est raccordée à des premières extrémités correspondantes (1050) des fils conducteurs (1010) de ladite première série de fils conducteurs (1010) et d'une manière telle que tous les fils conducteurs (1010) de ladite première série de fils conducteurs (1010) sont raccordés à des broches conductrices (1600) ;
procurer un deuxième cadre de montage (1001) englobant une deuxième série de fils conducteurs (1011), chacun possédant une première extrémité (1051) et une deuxième extrémité (1021) ;
raccorder à demeure ladite première extrémité (1051) de chaque fil conducteur dudit deuxième cadre de montage (1001) à des broches conductrices correspondantes parmi lesdites broches conductrices (1600) qui ne sont raccordées à aucun fil conducteur de ladite première série de fils conducteurs (1010) ; et
raccorder à demeure ladite deuxième extrémité (1021) de chaque fil conducteur de ladite deuxième série de fils conducteurs (1011) à des blocs de contact correspondants parmi lesdits blocs de contact, différents des blocs de contact raccordés à l'un quelconque des fils conducteurs de ladite première série de fils conducteurs (1010) ;
dans lequel
la première série de fils conducteurs (1010) et la deuxième série de fils conducteurs (1011) sont enlacées l'une dans l'autre.

2. Procédé selon la revendication 1, dans lequel ladite étape de raccordement à demeure comprend une étape de soudage pour mettre en oeuvre ledit raccordement à demeure.

3. Procédé selon la revendication 1 ou 2, dans lequel ladite au moins une structure de raccordement temporaire comprend une structure de raccordement temporaire qui raccorde les unes aux autres lesdites premières extrémités (1050) ou lesdites deuxièmes extrémités (1021) des fils conducteurs de ladite première série de fils conducteurs (1010), ladite structure de raccordement temporaire (1100, 1160) étant retirée lorsque ledit cadre de montage (1000) est monté dans ledit dispositif médical (610).

4. Procédé selon la revendication 3, comprenant en outre une autre structure de raccordement temporaire (1100, 1160) qui raccorde les unes aux autres les autres extrémités parmi lesdites premières extrémités (1050) ou les deuxièmes extrémités (1021) des fils conducteurs de ladite première série de fils conducteurs (1010), lesdites structures de raccordement temporaire (1100, 1160) étant retirées lorsque ledit cadre de montage (1000) est monté dans ledit dispositif médical (610).

5. Procédé selon la revendication 1, dans lequel lesdits blocs de contact auxquels est raccordée à demeure ladite deuxième extrémité (1020) des fils conducteurs de ladite première série de fils conducteurs (1010) sont prévus sur un alésage unique dudit dispositif médical (610) pour supporter une broche d'électrode; et/ou dans lequel lesdits blocs de contact auxquels est raccordée à demeure ladite deuxième extrémité (1020) des fils conducteurs de ladite première série de fils conducteurs (1010) sont distribués sur plus d'un alésage dudit dispositif médical (610) pour supporter plusieurs broches d'électrodes.

6. Procédé selon la revendication 1, dans lequel ledit premier cadre de montage (1000) englobe une première structure de raccordement temporaire (1100) qui raccorde les unes aux autres les premières extrémités (1050) des fils conducteurs de ladite première série de fils conducteurs (1010) et une deuxième structure de raccordement temporaire (1160) qui raccorde les unes aux autres les deuxièmes extrémités (1020) des fils conducteurs de ladite première série de fils conducteurs (1010), ladite première structure de raccordement (1100) et ladite deuxième structure de raccordement (1160) étant toutes deux retirées au cours du montage ; et dans lequel ledit deuxième cadre de montage (1001) englobe une troisième structure de raccordement temporaire (1101) qui raccorde les unes aux autres les premières extrémités (1051) des fils conducteurs de ladite deuxième série de fils conducteurs (1011) et une quatrième structure de raccordement temporaire (1161) qui raccorde les unes aux autres les deuxièmes extrémités (1021) des fils conducteurs de ladite deuxième série de fils conducteurs (1011), ladite troisième structure de raccordement (1101) et ladite quatrième structure de raccordement (1161) étant toutes deux retirées au cours du montage.
